# EUROPEAN PATENT SPECIFICATION

(11) **EP 3 809 980 B1**
(45) Date of publication and mention of the grant of the patent: **23.07.2025**
(21) Application number: 19732648.1
(22) Date of filing: 21.06.2019
(51) Int. Cl.: A61B 8/12, A61B 8/08, A61B 8/00, A61B 1/00, A61B 5/00

(54) **FORCE SENSING DEVICE, MEDICAL ENDODEVICE AND PROCESS OF USING SUCH ENDODEVICE IN VITRO**
KRAFTMESSVORRICHTUNG, MEDIZINISCHES ENDOGERÄT UND VERFAHREN ZUR VERWENDUNG EINES SOLCHEN ENDOGERÄTS IN VITRO
DISPOSITIF DE DÉTECTION DE FORCE, ENDODISPOSITIF MÉDICAL ET MÉTHODE D'UTILISATION D'UN TEL ENDODISPOSITIF IN VITRO

(30) Priority: 22.06.2018 CH 7932018
(43) Date of publication of application: 28.04.2021
(73) Proprietor: Universität Basel Vizerektorat Forschung, 4001 Basel (CH)
(72) Inventor: RAUTER, Georg, 5074 Eiken (CH); SUSIC, Ivan, 4056 Basel (CH)
(74) Representative: Latscha Schöllhorn Partner AG
(86) International application number: PCT/EP2019/066519
(87) International publication number: WO 2019/243597

(56) References cited:
- WO-A1-2016/125176
- US-A- 5 207 554
- US-A1- 2016 331 216
- US-A1- 2017 035 991

## Description

### Technical Field

The present invention relates to a force sensing device according to independent claim 1 and more particularly to a medical endodevice equipped with such a force sensing device and an in vitro process of detecting a type of tissue by means of such a medical endodevice.

Force sensing devices having a housing tip, a plurality of sensors stationarily spaced from each other and a spring element connecting the housing tip to the sensors, such that moving the housing tip and the sensors relative to each other causes a resilient deformation of the spring element, wherein the spring element is configured to arrange the housing tip in a zero position relative to the sensors by a spring force of the spring element, can be used in an endoscope or catheter for gathering information or data in addition to visual data obtained by a camera as it is conventionally provided in endoscopes.

### Background Art

For allowing minimal invasive interventions or analysis inside a human or animal body, it is known to use devices which are forwarded through a body lumen to a target location. There, suitable means or measures are applied. For example, the device can be equipped with a tool such as a drill, a saw, a laser source or the like which intervenes tissue at the target location. Or, the device can have a camera for obtaining optical information of the situation at the target location. For example, it is known to embody an endoscope appropriately, to forward the endoscope face through a body lumen and to apply the analysis and/or intervening means at the target location reached via the body lumen.

In recent years, the precision of devices like endoscopes and of the tools used with endoscopes has increased. For instance, in order to precisely controlling the endoscopes, robots are used which allow to provide sophisticated movements. Thereby, even though it was possible to have the robots to autonomously execute interventions, typically operators stay involved (robot guided interventions).

With respect to analyzing the target tissue or gathering data about the situation at the target location, it has been found in recent years that information in addition to the optical information provided by the camera can be helpful or required. In particular, it has been found that haptic information about the contact pressure between the endoscope or similar device and the body tissue or palpation information relating to deformation of endoscope and contact forces to tissue stiffness can be desired in many applications.

In this context, JP 2006-64465 A describes a sensor measuring omnidirectional contact forces of an endoscope or catheter front end. This sensor has a hemispheric tip connected to three strain gauges via a plurality of elastic plates or rings. When moving the tip relative to the strain gauges at least some of the strain gauges are contacted which induces a change in resistance of the affected strain gauges. The change of resistance is transformed into an electrical signal. By evaluating the electrical signal information about the pressure in terms of strength as well as direction can be gathered and evaluated.

A drawback of known endoscope tip sensors is that a force range covered is given by the setup of the sensor. For example, in the sensor described in JP 2006-64465 A the force range is predefined by the properties of the material of the elastic plates. However, the desired force range appropriate for a specific application may widely vary such that either plural different sensors have to be used or the sensor has to be de-assembled and provided with components suitable for the specific application.

In US 5,207,554 a supporting device is described which comprises a first member which supports a springy means for resiliently supporting a second member. The supporting device comprises a detection means for detecting displacement of the springy means with respect to the first member in accordance with the movement of the second member and a biasing means which applies a force to the springy means from the same direction as or from a direction opposite to that of the displacement for changing the elasticity of the springy means in accordance with the movement of the second member.

WO 2016/125176 A1 discloses a limiter configured to be coupled to a medical device and methods thereof. The limiter is configured to provide a preventive action and/or a precautionary action, responsively to a predefined excessive contact of the medical device with a tissue. According to some embodiments, the preventative action is at least one selected from: an alert to a user, energy conversion in order to control the contact, and prevention of the medical device from further force application and/or further contact with the tissue.

US 2017/035991 A1 concerns a force sensing catheter having a proximal segment containing a proximal hub, a distal segment containing a distal hub, and a spring segment that extends from the proximal segment to the distal segment. The spring segment comprises a plurality of struts connected to both of the proximal and distal hubs. Each strut includes a preformed bend. The struts mechanically support the distal segment in a base orientation with respect to the proximal segment, flex at the preformed bends when the distal segment moves relative to the proximal segment in response to a force, and resiliently return the distal segment to the base orientation once the force has been removed. The catheter further comprises a plurality of sensors configured to output signals indicative of relative movement between the proximal and distal segments for determining a magnitude and direction of the force.

Therefore, there is a need for a force sensing device allowing a flexible operation in various different applications and, at the same time, for accurately providing information about a tilting movement. Also, there is a need for an endodevice and a tissue analysis process using such a force sensing device.

### Disclosure of the Invention

According to the invention this need is settled by a force sensing device as it is defined by the features of independent claim 1, by a medical endodevice as it is defined by the features of independent claim 10, and by a process as it is defined by the features of independent claim 14. Preferred embodiments are subject of the dependent claims.

In one aspect, the invention is a force sensing device comprising a housing tip, a plurality of sensors structure. The sensors are stationarily spaced from each other. The force sensing device further comprises a spring element connecting the housing tip to the sensors such that moving the housing tip and the sensors relative to each other causes a resilient deformation of the spring element; wherein the spring element is configured to arrange the housing tip in a zero position relative to the sensors by a spring force of the spring element. The force sensing device further comprises a spring tensioning structure configured to adapt the spring force of the spring element in order that the range of forces to be detectable by the force sensing device can be adjusted, wherein the force sensing device can efficiently be prepared for different conditions and applications.

The term "stationary" as used herein can relate to a fixed spatial arrangement. For example, the sensors can be stationarily spaced from each other by being fixedly mounted distant from each other and being immovable relative to each other.

The number of sensors can be chosen in accordance with the required needs of the intended application of the force sensing device. For allowing detection of three dimensional movements of the housing tip, preferably at least three sensors are provided.

The spring element connects the housing tip to the sensors such that moving the housing tip and the sensors relative to each other causes a resilient deformation of the spring element. The spring element is configured to arrange the housing tip in a zero position relative to the sensors by its spring force. Thereby, the movement of the housing tip and the sensors relative to each other can also be referred to as tip bending or tilting.

The spring element can be directly or, particularly, indirectly connected to the sensors. Advantageously, the sensors are mounted to a support structure like a support plate as described below and the spring element is mounted to the support structure. Thereby, a portion of the spring element can be stationary to the sensors. The spring element can be a single part construction or a multi parts construction.

The spring tensioning structure is configured to adapt the spring force of the spring element. Thereby, the range of forces to be detectable by the force sensing device can be adjusted. For example, the force range can be from 0 Newton (N) to 8N. Like this, the device can be efficiently set up for a specific application without requiring any de- and re-assembling of the device or without having plural differently embodied devices at hand. For example, it can be prevented that the spring element has to be replaced in order to vary the spring force. Thus, the force sensing device can efficiently be used in or prepared for different conditions or applications.

More specifically, the spring tensioning structure can be an active spring tensioning arrangement which allows for actively adjusting the spring force. Thereby, the spring tensioning structure can be arranged to stepwise or continuously vary the spring force over a certain range.

Furthermore, the arrangement of the device according to the invention allows for detecting any movement of the housing tip relative to the sensors such as a tilting movement. Like this, not only bending or tilting of the force sensing devices can be detected but also a movement of the device, e.g., along a tissue of patient.

Thus, the force sensing device according to the invention allows for a convenient operation in various different applications and, at the same time, for accurately providing information about a movement of the housing tip relative to the sensors.

Preferably, the sensors of the force sensing device are unidirectional sensors. The term "unidirectional" in this connection relates to a configuration for measuring or detecting a force or movement quasi exclusively in one single direction. Unidirectional sensors typically are embodied to generate a signal when a force is acting on the sensor in a predefined direction or a movements is detected in a predefined direction. For example, unidirectional force sensors can be equipped with a button which has to be pressed in order that such signal is generated. By using such unidirectional sensors it can be achieved that movements of the housing tip can efficiently be detected or measured. In particular, implications of other movements or forces not directly related to the movement of the housing tip can be reduced or eliminated.

The sensors can be any sensors suitable for detecting a movement of the housing tip. For example, the sensors can be optical sensors detecting the relative position of a landmark on the housing tip and a landmark associated to the sensors. Or the sensors can comprise acceleration sensor or gyroscopes mounted to the part of the device moving relative to each other. The relative movements and applied forces can then be detected by evaluating the signals provided by these sensors and eventually properties of the spring element such as its spring force. However, preferably, the sensors of the force sensing device are force sensors. Such force sensors allow for a very efficient, accurate, and reliable determination of movements and forces involved when tilting or bending the housing tip.

Thereby, the force sensing device preferably comprises a plurality of sensor actuators, each associated to one of the force sensors, wherein the sensor actuators are coupled to the housing tip such that moving the housing tip relative to the force sensors out of the zero position causes at least one of the sensor actuators to act on the associated force sensor. Such arrangement of sensor actuators allows for an accurate actuation of the force sensors with a comparably simple and flexible set up.

For coupling the sensor actuators to the housing tip, they preferably are stationarily mounted to the housing tip. Such an arrangement of the actuators and the tip allow for transferring any movement of the tip directly to the actuators which, in turn, act on the force sensors accordingly. Like this, an efficient and accurate sensor activation upon a movement of the tip can be achieved.

The force sensing device can be embodied in a free floating design. This is, the housing tip is coupled to the sensors in a manner that at least to a certain extent solely the spring element provides a connection between those components. For example, the spring element can be directly or indirectly mounted to the housing tip and directly or indirectly to the sensors, wherein no other element connects the housing tip with the sensors. Such design allows for measuring forces in positive and negative directions such as, e.g., pressure and tension, and/or shear forces in two opposed senses per direction.

Preferably, each of the sensor actuators is adjustable in its extension towards the associated force sensor. The term "extension" in this connection can particularly relate to a dimension between the housing tip and the force sensors. In embodiments where the sensor actuators are elongated bodies, the extension can be a length of the respective body.

By having such flexible extensions, the sensor actuators may be adjusted to more or less strongly act on the force sensors. Also, it allows to adjust the sensor actuators in correspondence with the adjustment of the spring element such that an appropriate contact between the actuators and the sensors can be predefined for any predefined force range.

Preferably, the force sensing device comprises a preferably flexible sensor support plate on which the sensors are mounted. Such a sensor support plate allows for conveniently and securely arranging the sensors in a stationary manner spaced from each other. In particular, it allows for well predefining the spatial relationship between the sensors as well as between the spring element and the sensors. The sensor support plate can be flat or three dimensionally structured or shaped. When being embodied in a flexible fashion, the sensor support plate allows for increasing the extent of movement applied towards the sensors.

Thereby, the sensor support plate preferably is a printed circuit board (PCB). A PCB typically mechanically supports and electrically connects electronic components arranged thereon. It may be equipped with conductive tracks, pads and other features etched from one or more sheet layers of copper laminated onto and/or between sheet layers of a non-conductive substrate. Components are generally soldered onto the PCB to both electrically connect and mechanically fasten them to it. Thus, by using a PCB as sensor support plate, the sensors as well as, optionally, other electronic components can conveniently be held and wired.

Generally, the sensor support plate can have any shape suitable for an appropriate support of the sensors. The sensors can be mounted to one common surface of the support plate, wherein this surface can be flat or structured. For example, the surface can have one or more inclined sections which allow for positioning the sensors in a preferred orientation. E.g., in embodiments where the sensors are force sensors, it can be beneficial to incline the sensors in order to better receiving the respective forces or elements such as the actuators.

The sensor support plate is essentially ring-shaped. Like this, a central access hole can centrally be provided through which the housing tip or a through hole therein can conveniently be accessed. This can particularly be beneficial when the force sensing device is used in an endodevice such as an endoscope, a catheter or the like.

Thereby, the sensors preferably are regularly distributed about the central hole of the ring-shaped support plate. In embodiments where there are exactly three sensors provided, advantageously they are arranged at a 120° angle to each other on the support plate. Like this, three dimensional movements of the housing tip relative to the sensors can efficiently be evaluated.

Preferably, the spring tensioning structure comprises a screw member and a screw socket such that by screwing the screw member into or out of the screw socket the spring force of the spring element is adapted. With such an arrangement the spring element can be more or less pre-compressed such that its resistance can be predefined. This allows for efficiently adjusting the sensitivity of the force sensing device and, particularly, its force range.

Preferably, the spring element comprises a helical spring being advantageously stationarily connected to the sensors at one of its longitudinal ends and stationarily connected to the housing tip at the other one of its longitudinal end. The spring element can also have plural helical springs which can be arranged parallel to each other or extending through each other. Such arrangement having helical spring(s) allows for efficiently embodying a flexibly adjustable spring element.

Thereby, the screw member preferably extends along the helical spring. Thereby, the term "along" also encompasses arrangements where the screw member extends through the spring element, e.g. though a helical spring. Like this, the helical spring can efficiently and precisely adjusted, i.e., pre-tensioned.

Preferably, the housing tip is essentially dome shaped. The term "dome" as used herein can relate to a partial spherical or sphere-like form such as a hemisphere or the like. It can also comprise a more pointed shape than a geometrical (hemi)sphere. Such a housing tip allows for efficiently equipping an endodevice with the force sensing device as its tip or face.

Preferably, the force sensing device comprises a safety structure limiting a maximum movement of the housing tip and the sensors relative to each other. With such a safety structure it can be prevented that the sensors are damaged by the sensor actuators too strongly contacting the sensors. In particular, by limiting the maximum movement it can be achieved that a movement of the sensor actuators is stopped before they too hardly hit the sensors. Such arrangement allows for a secure usage of the force sensing device such that its lifetime can be increased.

Preferably, the force sensing device comprises a guiding structure predefining possible movements of the housing tip and the sensors relative to each other. Thereby, the guiding structure preferably is configured to prevent rotational movements of the housing tip and the sensors relative to each other. Particularly, rotational movements about a longitudinal axis of the force sensing device are prevented. The term "longitudinal axis" in this connection can relate to an axis centrally extending from a proximal end to a distal end of the device. It can be a symmetry axis of the device wherein such symmetry axis can be involved not only in strictly symmetric set ups of the device but also in embodiments where the outer shape of the device is more or less symmetric.

Also, the guiding structure preferably is configured to limit lateral shifting, as well as optionally a vertical movement and bending, of the housing tip and the sensors relative to each other. In particular, the guiding structure preferably is configured to limit the movements of housing tip and the sensors relative to each other to five degrees of freedom. As mentioned above, in a preferred embodiment the sixth degree of freedom being rotation about the longitudinal axis is eliminated. Like this, it can be achieved that the housing tip can exclusively be laterally shifted (2 degrees of freedom), vertically moved (1 degree of freedom), and bent or tilted (2 degrees of freedom). This allows for significantly improving the accuracy of measuring or determining the applied force to the housing tip. For example, disturbing movements or forces less relevant for the tip bending or tilting can be efficiently eliminated by such a guiding structure.

In a preferred embodiment, the safety structure and the guiding structure are embodied by at least one projection stationary to one of the housing tip or the sensors and at least one corresponding recess stationary to the other one of the housing tip and the sensors, the at last one recess receiving the at least one projection. Thereby, the force sensing device can also be equipped with plural such projections and plural such recesses. In particular, it can have a sufficient number of each in order to efficiently secure and guide the housing tip in all tilting directions. Advantageously, an uneven number of each, such as five or the like, is provided since in this way, centering of the housing tip can efficiently be achieved while it might get more easily stuck when an even number of each is implemented. Such projection and recess allow for efficiently implementing the safety and guiding structures in one.

In one aspect of the present disclosure, the force sensing device can be embodied with any of the guiding structures mentioned above but without a spring tensioning structure.

Preferably, the force sensing device has a distal end and a proximal end, wherein the sensors are configured to sense a force along an axis extending between the distal and proximal ends of the force sensing device. The term "distal" can relate to a direction intended to be oriented towards the target tissue. Thus, the distal end can be a front end of the device. Analogously, the term "proximal" as used herein can relate to a direction away from the target tissue or towards an operator or a machine such as a robot controlling the device. By having such unidirectional sensors, tilting of the housing tip can efficiently be gathered in extent as well as in change. This allows for qualitatively and quantitatively determining the movement of the housing tip.

In another aspect, the invention is a medical endodevice comprising an elongated liaising structure with a distal end as well as a force sensing device as described above. The force sensing device is mounted to the distal end of the elongated liaising structure such that the housing tip forms a tip of the medical endodevice, wherein the liasing structure comprises an articulated portion. In particular, the housing tip of the force sensing device forms a front or distal end of the endodevice.

The term "proximal" as used in connection with the endodevice or its liaising structure can relate to a direction towards an operator of the endodevice or a machine such as a robot controlling the endodevice. Analogously, the term "distal" can relate to a direction away from the operator or machine.

The term "endodevice" in connection with the invention relates to a device which is arranged to be introduced into a body or body lumen and to be advanced through the body or body lumen to a target location where an action such as an intervention is to be executed. Thereby, the term "in the body" or "inside the body" can mean any location in the human or animal body and particularly a quasi embedded location which is not directly accessible from the outside. For example, in the body can mean in between different tissues of the body, such as in between a bone and its surrounding tissue, or inside a body lumen. The term "body lumen" can relate to an inside space of a tubular structure in a human or animal body or to a cavity inside the human or animal body. For example, the body lumen can be a vascular vessel, such as a vein or an artery or a coronary or intracranial vessel or a heart valve, or a tract of a gastrointestinal organ such as stomach or colon, or a region of urinary collecting ducts or of renal tubes, or an interior space of joint, or a mouth or ear, or a combination thereof. The endodevice can be or comprise a rigid or particularly a flexible endoscope, a catheter, a laparoscope, a colonoscope or a similar arrangement.

The endodevice according to the invention allows for efficiently achieving the effects and benefits of the force sensing device according to the invention and its preferred embodiments described above. Particularly, it allows to use the force sensing device in a variety of medical applications. Specifically, the endodevice allows for providing palpation information which physically can not be obtained since the target tissue is inside a human or animal body. Thus, the endodevice allows for giving the operator or practitioner information as if he could touch the tissue at the target tissue, which is not available without, e.g. surgically, accessing the target tissue.

Preferably, the elongated liaising structure comprises an articulated portion. Such articulated portion allows for forwarding the endodevice in or along a comparably complicated structure in the body such as along a vessel including curves and the like. Thereby, the articulated portion of the elongated liaising structure preferably comprises a plurality of bodies and angle sensors. Each of those bodies is tiltably connected to another one of the bodies and each angle sensor is configured to determine an angle between two connected bodies. In one possible embodiment, each body is connected to another one of the bodies to be tiltable exclusively about one single axis. By means of such an articulated portion a cascaded or chain-like arrangement can be formed in which position and orientation of each chain link can be exactly determined such that the course of the chain can precisely be known. This allows for accurately determining the location and orientation of the force sensing device within a human or animal body can efficiently be determined. In particular, by such arrangement an imaging process, e.g. by a magnetic resonance imaging apparatus or the like, for determining the location of the tip of the endodevice can be prevented.

The elongated liaising structure comprises a force/torque sensor unit proximally spaced from the distal end. The term "force/torque sensor" in this connection relates to an arrangement allowing to sense a force and/or a torque. Thus, it may be or comprise a force sensor, a torque sensor or any combination of force and torque sensors.

Such a force/torque sensor unit allows for improving the quality of the signals provided by the endodevice. In particular, it allows for comparing the signals provided by the force sensing device, i.e. the tip, to the force/torque sensor unit such that disturbing forces, pressures, movements or other disturbing effects along the elongated liaising structure can be determined. For example, a friction or interaction force acting along the elongated liaising structure of the endodevice due to a forwarding movement of the complete endodevice can be detected by deducing the force detected at the tip by the force sensing device from the forces detected by the force/torque sensor unit on the proximal side (for the deduction, the forces need to be transformed to the same coordinate system). So if friction or interaction forces are high, the practitioner can be warned that the forces along the endodevice's elongated liaising structure may be dangerous for harming surrounding tissue.

In some embodiments of the endodevice being adapted for specific applications the liaising structure can also be equipped with plural identical or similar force/torque sensor units.

It is to note that, even though the embodiments of the endodevice having the force/torque sensor unit are described herein in connection with the force sensing device according to the invention, it is also beneficial to provide an endodevice having a conventional force sensing device at its tip with a proximally spaced force/torque sensor unit as described above and below. In particular, such an embodiment would also allow to deduct the force detected at the tip from the forces detected by the force/torque sensor unit on the proximal side, when conventional force sensing devices are used at the tip.

In embodiments with liaising structures having an articulated portion, the articulated portion of the elongated liaising structure preferably has a distal side and a proximal end side, wherein the force sensing device is located distally from the articulated portion and the sensor unit being located proximally from the articulated portion. Such a force/torque sensor unit allows for determining an orientation of the force sensed by the force sensors of the force sensing device. Knowing this orientation can be crucial when applying the endodevice. In particular, the orientation of the tip can be decisive for differentiating between various kind of movements such as between pushing or pulling movements. For example, the force sensing device provides the same force sensor signals in case where the endodevice is straight and forwardly pushed as where the endodevice is turned by 180° and backwardly pulled. In these two situations the force/torque sensor unit provides opposite force signals. Thus, by having both, the signals from the force sensors of the force sensing device as well as the signals from the force/torque sensor unit, the orientation of the sensed force can accurately be determined.

Thereby, the force sensor unit of the elongated liaising structure preferably is similarly embodied as the force sensing device. In particularly, it can comprise (i) three unidirectional shaft force sensors stationarily spaced from each other, (ii) three shaft sensor actuators, each associated to one of the three shaft force sensors; and (iii) a shaft spring element connecting the articulated portion of the elongated liaising structure to the three shaft force sensors such that moving the articulated portion and the shaft force sensors relative to each other causes a resilient deformation of the shaft spring element, wherein the shaft spring element is configured to arrange the articulated portion in a zero position relative to the shaft force sensors by a spring force, and the shaft sensor actuators are coupled to the articulated portion such that moving the articulated portion relative to the shaft force sensors out of the zero position causes at least one of the shaft sensor actuators to act on the associated shaft force sensor.

Preferably, the medical endodevice comprises a camera located at the tip of the medical endodevice. Such a camera allows for the endodevice being a similar device as an endoscope. More specifically, it allows for enriching the force information obtained by the force sensing device with visual information gathered by the camera.

The force sensing device according to the invention and the endodevice according to the invention can be used in various different applications. Whereas they can be particularly advantageous in medical applications, they can also be used in industrial or other research applications. In particular, they can be used in any application where a stiffness or other surface or palpation property of a material is to be determined, particularly, in an inside of a body or element which has a reduced accessibility.

In a further other aspect, the invention is an in vitro process of detecting a type of tissue. The process comprises the steps of: obtaining a medical endodevice as described above, positioning the tip of the medical endodevice at a target surface, moving the tip of the medical endodevice along the target tissue, obtaining sensor data generated by the sensors of the force sensing device of the medical endodevice, and determining a stiffness of the tissue at the surface by evaluating the obtained sensor data of the sensors of the force sensing device.

By the process according to the invention, the endodevice according to the invention and its preferred embodiments can efficiently be used for detecting what type or quality of tissue is involved. In particular, the process allows for using palpation information to detect the type of tissue similar as if the tissue was freely accessible to the hands of a practitioner. For example, by implementing the endodevice according to the invention the process makes it possible to obtain information about a stiffness of the tissue and, more specifically, about a change in stiffness of the touched or contacted tissue. Like this, e.g., cancerous tissue can be detected inside the body in an efficient and reliable manner. Thereby, a change of movement in relation to the detected force or pressure can be important for deriving the stiffness of the tissue.

Preferably, the process further comprises the steps of obtaining sensor data generated by the force/torque sensor unit of the endodevice, and determining interactions along the endodevice by evaluating the sensor data of the force/torque sensor unit. Tthe process further preferably comprises a step of calculating disturbances acting on the endodevice by comparing the sensor data of the force/torque sensor unit with the sensor data of the sensors of the force sensing device. Such process allows for increasing the quality of the palpation information gathered.

In particular, the process can be a purely in vitro process.

### Brief Description of the Drawings

The force sensing device according to the invention, the medical endodevice according to the invention and the process according to the invention are described in more detail herein below by way of exemplary embodiments and with reference to the attached drawings, in which:
- Fig. 1: shows a perspective exploded view of a first embodiment of a force sensing device according to the invention;
- Fig. 2: shows a first side view of the force sensing device of Fig. 1;
- Fig. 3: shows a second side view of the force sensing device of Fig. 1;
- Fig. 4: shows a cross sectional view along the line A-A of the force sensing device of Fig. 2;
- Fig. 5: shows a cross sectional view along the line B-B of the force sensing device of Fig. 3;
- Fig. 6: shows a perspective exploded view of a second embodiment of a force sensing device according to the invention;
- Fig. 7: shows a side view of the force sensing device of Fig. 6;
- Fig. 8: shows a cross sectional view along the line A-A of the force sensing device of Fig. 7;
- Fig. 9: shows a perspective exploded view of a third embodiment of a force sensing device according to the invention;
- Fig. 10: shows a side view of the force sensing device of Fig. 9; and
- Fig. 11: shows a cross sectional view along the line A-A of the force sensing device of Fig. 10.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes includes various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

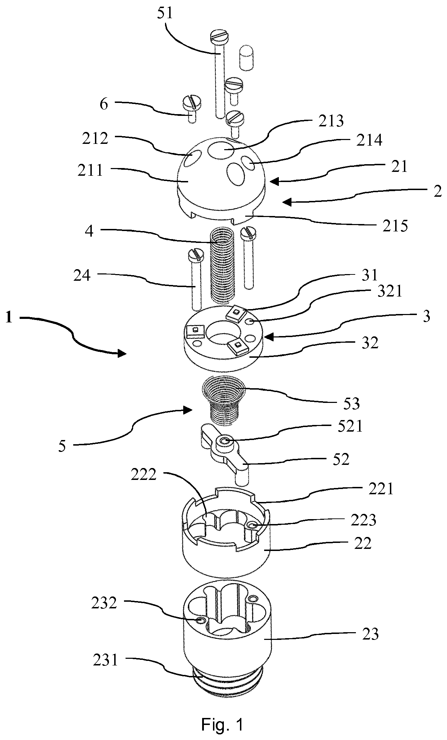

Fig. 1 shows an exploded view of a first embodiment of a force sensing device 1 according to the invention to be implemented in a first embodiment of a medical endodevice according to the invention. The force sensing device 1 comprises a multi part housing 2 with a tip 21, an intermediate ring 22, a base 23 and two mounting screws 24. The tip 21 is essentially dome shaped and has a hemisphere portion 211 equipped with a central tensioner receptacle 213 having an opening, three actuator receptacles 212 having openings, and a camera opening 214. At its bottom end, the tip 21 has four downwardly extending axial projections 215 which are regularly distributed about a circumference of the bottom end.

The intermediate ring 22 has an internal geometry forming a socket bar receptacle 222 and two lateral screw through holes 223. Further, at its top end it is equipped with four regularly distributed axial recesses 221 which are upwardly open. The recesses 221 are shaped and positioned in correspondence with the projections 215 of the tip 21. The base 23 has two lateral screw sockets 232 at its top end for receiving the mounting screws 24 and a threaded connector 231 at its bottom end for being mounted to a liaising structure of the medical endodevice such that the force sensing device 1 establishes the tip of the endodevice.

The force sensing device 1 further comprises a sensor assembly 3, a vertically extending helical spring 4 of a spring element, a spring tensioning structure 5 and three actuator screws 6 as sensor actuators. The sensor assembly 3 comprises a printed circuit board (PCB) ring 32 with a central bore as support plate. On its top surface, the PCB ring 32 is equipped with three unidirectional force sensors 31 which are distributed in 120° angels towards each other on the PCB ring 32. Further, the PCB ring 32 has two lateral opposing through holes 321.

The spring tensioning structure 5 has a vertically extending tensioning screw 51, a socket bar 52 with a central screw socket 521 and a helical counter spring 53. The counter spring 53 is downwardly tapering, wherein its bottom end is dimensioned to lie on the socket bar 52 beneath the screw socket 521.

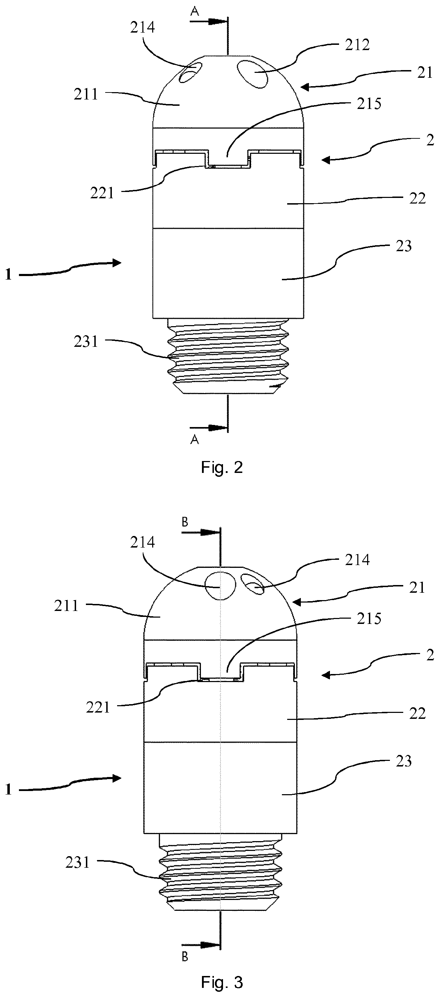

In Figs. 2 and 3 the force sensing device 1 is sidewise shown in an assembled state, wherein in Fig. 2 it is turned by 90° about its vertical central axis compared to Fig. 3. As can be seen, the housing 2 surrounds and covers all other components of the force sensing device 1. The projections 215 of the tip 21 are positioned inside the recesses 221 of the intermediate ring 22. Thereby, the top end of the intermediate ring 22 and the bottom end of the tip 21 contact free inter-engage in a teeth like fashion. The openings of the actuator receptacles 212 and of the camera opening 214 are provided in the hemisphere 211 of the tip 21.

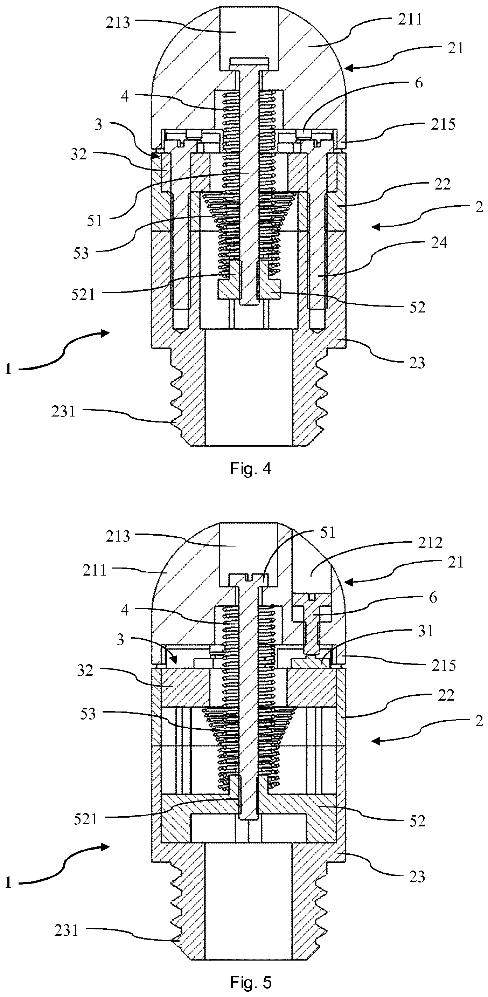

Fig. 4 shows a cross section of the force sensing device 1. Thereby, it can be seen that the PCB ring 32, the intermediate ring 22 and the base 23 are rigidly connected to each other by the mounting screws 24, which extend top down through the through holes 321 of the PCB ring 32 and of the intermediate ring 22, and are screwed into the screw sockets 232 of the base 23. Like this, the sensor assembly 3 is stationary to the intermediate ring 22 and the base 23.

The tip 21 of the housing 2 is mounted to the base 23 via the tensioning screw 51 extending through the helical spring 4 and being screwed into the screw socket 521 of the socket bar 52. The socket bar 52 is pressed to the base 52 by the counter spring 53 and the helical spring 4, which act on the counter bar 52 at its lower end and to the PCB ring 32 at its top end. The helical spring 4 extends centrally through the PCB ring 32 and through the counter spring 53. On its top side the helical spring 4 abuts from below at the tensioner receptacle 213 and at its bottom end at the top surface of the screw socket 521 of the socket bar 52. Thus, the more the tensioning screw 51 is screwed into the screw socket 521 the more the helical spring 4 is tensioned.

Since the tip 21 is connected to the base 23 via the helical spring 4, it can be moved to a certain extent in relation to the base 23 and, thus, the intermediate ring 22 and the PCB ring 32. Thereby, the projections 215 and recesses 221 limit the movement such that they define a maximum movability of the tip 21. Thus, the projections 215 and recesses 221 form safety and guiding structures of the force sensing device 1. Furthermore, the extent of tensioning the helical spring 4 by the tensioning screw 51 defines a resistance of the tip 21 with respect to movements. Like this, force sensibility of the force sensing device 1 can be defined.

In Fig. 5 another cross section of the force sensing device is shown. There, it can particularly be seen that each of the actuator screws 6 is associated top down to one of the force sensors 31. The force sensors 31 have an upwardly extending button which is adjacent to the lower end of the associated actuator screw 6. A length of the actuator screws 6 or their dimensions towards the force sensors 31 can be adjusted by screwing the actuator screws 6 more or less into the actuator receptacles 212. Thus, the actuator screws 6 can be adapted in accordance with the tensioning of the helical spring 4. In particular, their dimension can be adapted such that they are adjacent to the force sensors 31 without activating them when the helical spring 4 holds the tip 21 in an upright zero position. When the tip 21 is tilted or otherwise moved, for example by a tissue abutting the outer surface of the tip 21, the force sensor(s) 31 in the direction where the tip 21 is tilted are activated by pressing the button(s) more and more into the force sensor(s) 31.

The force sensing device 1 can be provided as the tip of the medical endodevice. This, in turn, can be used in a process of differentiating tissue types and detect contact forces (shear and normal forces). For this, the tip 21 of the medical endodevice is introduced into a body cavity and positioned at a target tissue or surface. Then, it is moved along the target tissue such that sensor data generated by the force sensors 31 of the force sensing device 1 of the medical endodevice are generated. By evaluating this sensor data the stiffness of the target tissue is determined (normal and shear components can be determined).

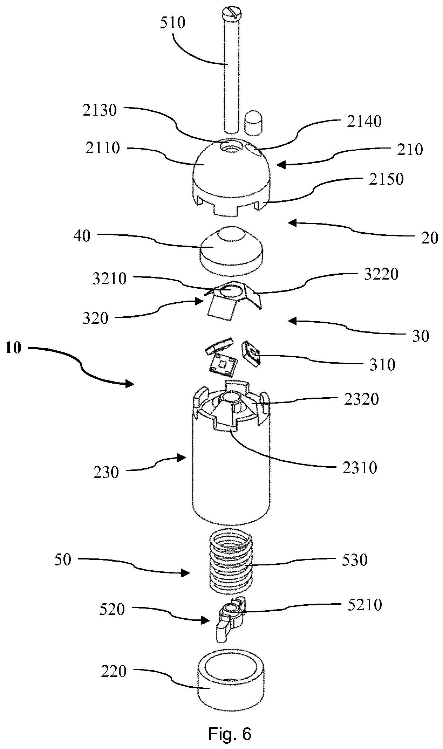

Fig. 6 shows an exploded view of a second embodiment of a force sensing device 10 according to the invention to be implemented in a second embodiment of a medical endodevice according to the invention. The force sensing device 10 comprises a multi part housing 20 with a tip 210, and a base 230. The tip 210 is essentially dome-shaped and has a hemisphere portion 2110 equipped with a central tensioner receptacle 2130 having an opening and a camera opening 2140. At its bottom end, the tip 210 has five downwardly extending axial projections 2150 which are regularly distributed about a circumference of the bottom end.

The base 230 is essentially cylindrical and has an internal geometry forming three inclined sensor abutting surfaces 2320. Further, at its top end it is equipped with five regularly distributed axial recesses 2310 which are upwardly open. The recesses 2310 are shaped and positioned in correspondence with the projections 2150 of the tip 210.

The force sensing device 10 further comprises a sensor assembly 30, a conical elastic body 40 of a spring element and a spring tensioning structure 50 having an axial vertical spring tensioning screw 510. The sensor assembly 30 comprises a flexible PCB 320 with a central bore portion 3210 and three inclined sensor carrier lamellas 3220 regularly extending from the central pore portion 3210 such that they are distributed in 120° angels towards each other. On their bottom surfaces, each of the sensor carrier lamellas 3220 is equipped with one unidirectional force sensor 310. The central bore portion 3210 is equipped with a central bore which is dimensioned to receive the spring tensioning screw 510 with clearance.

In addition to the spring tensioning screw 510, the spring tensioning structure 50 comprises a socket bar 520 with a central screw socket 5210, a helical counter spring 530 and a spring fixing ring 220. The counter spring 530 has a bottom end dimensioned to lie on the socket bar 520 beneath the screw socket 5210. The spring tensioning structure 50 predefines an upright zero position of the force sensing device 10.

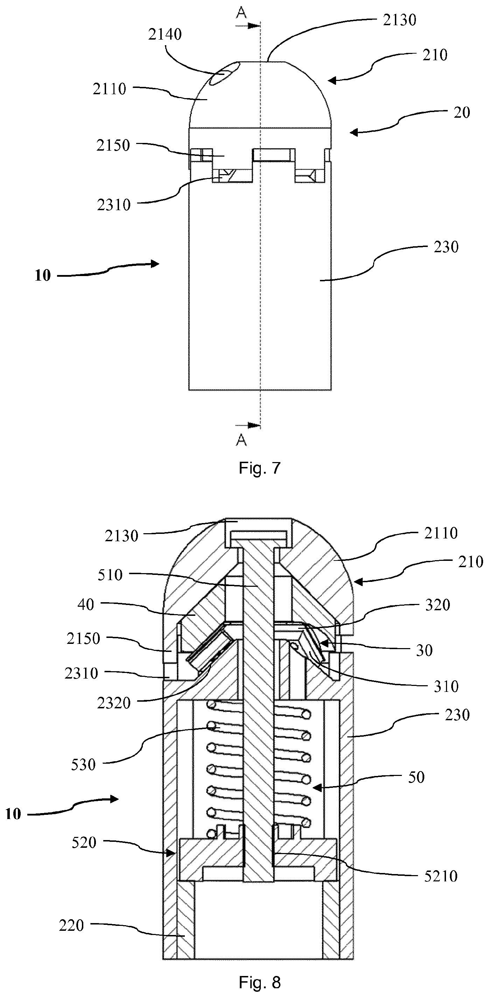

In Fig. 7 the force sensing device 10 is shown in an assembled state from a side in its upright zero position. As can be seen, the housing 20 surrounds and covers all other components of the force sensing device 10. The projections 2150 of the tip 210 are positioned inside the recesses 2310 of the base 230. Thereby, the top end of the base 230 and the bottom end of the tip 210 contact free inter-engage in a teeth like fashion. The camera opening 2140 is provided in the hemisphere 2110 of the tip 210.

Fig. 8 shows a cross section of the force sensing device 10 along the line A-A depicted in Fig. 7. Thereby, it can be seen that the sensor abutting surfaces 2320 are inclined. More specifically, the for an angle of about 45° relative to an axis of the force sensing device 10 or to the spring tensioning screw 510. Depending on the specific application and the sensitivity aimed to be achieved in a particular direction this angle can be adapted. Correspondingly, the sensor carrier lamellas 3220 of the PCB 320 and the lower surface of the elastic body 40 are also inclined in the same manner. The force sensors 310 are downwardly oriented towards the sensor abutting surfaces 2320.

The tip 210 of the housing 20 is mounted to the base 230 via the tensioning screw 510 extending through the elastic body 40 and being screwed into the screw socket 5210 of the socket bar 520. The socket bar 520 is clamped in between the counter spring 530 and the fixing ring 220. The elastic body 40 is in connection with the sensor assembly 30 at its lower end and with the tip 210 at its top end. Thus, the more the tensioning screw 510 is screwed into the screw socket 5210, the more the elastic body 40 and the counter spring 530 are compressed and thereby tensioned. Hereby, the tip 210 is setup in a free floating way supported by pretensioned spring elements, i.e. the elastic body 40 and the counter spring 530, to receive forces in positive and negative directions for any of the force sensing directions.

Since the tip 210 is connected to the base 230 in quasi free floating manner via the elastic body 40, it can be moved to a certain extent in relation to the base 230. Thereby, the projections 2150 and recesses 2310 limit the movement such that they define a maximum movability of the tip 210. Thus, the projections 2150 and recesses 2310 form safety and guiding structures of the force sensing device 10. Furthermore, the extent of tensioning the elastic body 40 by the tensioning screw 510 defines a resistance of the tip 210 with respect to movements such as tilting, pulling, pushing or the like. Like this, force sensibility of the force sensing device 10 can be variably defined. Due to the flexibility of the PCB 320 a movement or tilting of the tip relative base 230 results in a smoothened pressure applied to the force sensors 310.

The force sensing device 10 can be provided as the tip of the medical endodevice. This, in turn, can be used in a process of differentiating tissue types and detect contact forces (shear and normal forces) as described above.

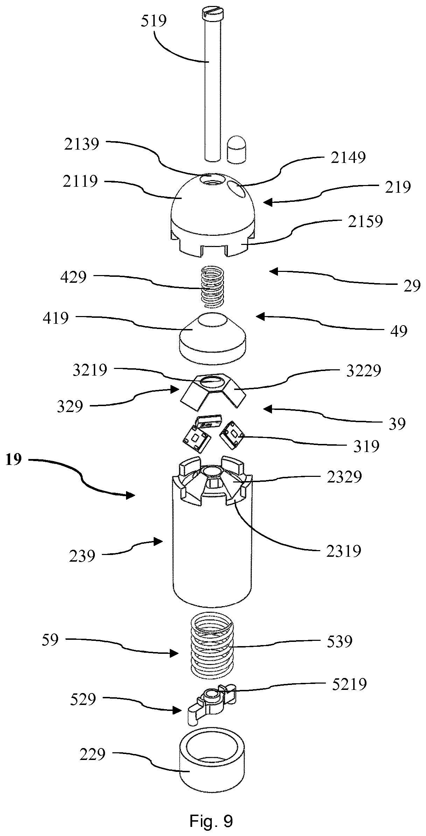

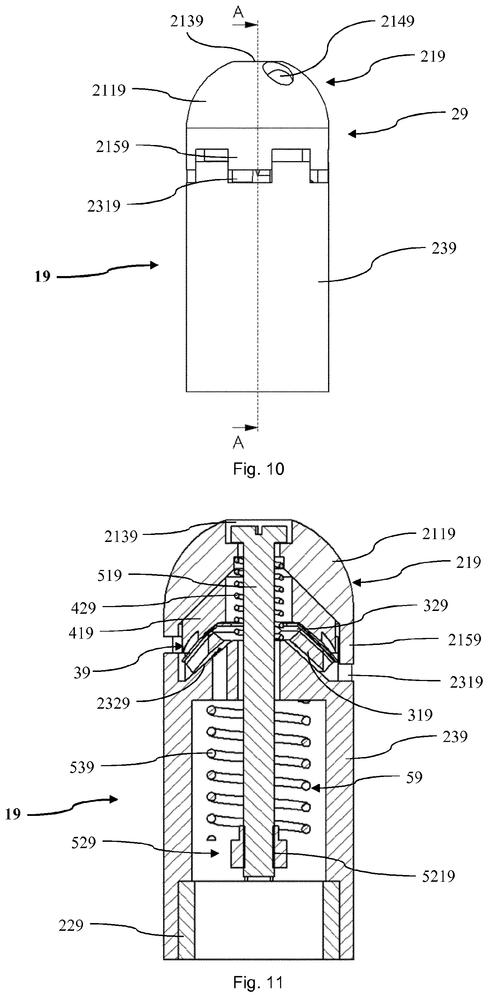

Fig. 9, Fig. 10 and Fig. 11 show an exploded view, a side view and a cross-sectional view of a third embodiment of a force sensing device 19 according to the invention to be implemented in a third embodiment of a medical endodevice according to the invention. The force sensing device 19 is similar to the second embodiment shown in Figs. 6, 7 and 8. Where not described to be different in the following, the third embodiment of the force sensing device 19 is essentially identical to the second embodiment of the force sensing device 10 and it is referred to the respective description of Figs. 6, 7 and 8 above.

Similar as the second force sensing device 10, the third force sensing device 19 comprises a multi part housing 29 with a tip 219 having a hemisphere portion 2119, a tensioner receptacle 2139 and a camera opening 2149, a base 239 having three inclined sensor abutting surfaces 2320 and five axial recesses 2310, and five downwardly extending axial projections 2159. The force sensing device 19 further comprises a sensor assembly 39 having three force sensors 319 and a flexible PCB 329 with a central bore portion 3219 and three inclined sensor carrier lamellas 3229, and a spring tensioning structure 59 having an axial vertical spring tensioning screw 519, a socket bar 529 with a central screw socket 5219, a helical counter spring 539 and a spring fixing ring 229.

Different from the second force sensing device 10, the third force sensing device 19 comprises a two part elastic element 49. More specifically, the elastic element 49 comprises a conical elastic body 419 and a helical tip spring 429. The tip spring 429 works together with the counter spring 539, to allow easier adaptation of the initial force range of the force sensing device 19 and its sensitivity. The tip spring 429 is positioned between the hemisphere portion 2119 of the tip 219 and the base 239. More specifically, it is arranged to force the tip 219 and the base 239 into a zero position predefined by the spring tensioning structure 59. The tip spring 429 is aligned to the elastic body 419, such that after a force is released, the tip spring 429 assists the force converting structure to get back to the initial or zero position faster. In particular, it can assist the elastic body 419 to efficiently get back its original shape or form once the force is released from the tip 219. While the force range of the three force sensors 319 stays the same or is given, the addition of the tip spring 429 allows adapting the force range of the force sensing device as a whole independent of the force range of the three force sensors 319. So, while the range of deformation of the elastic body 419 remains the same for any desired force range of the force sensing device 19, the tip spring 429 will ensure the transfer of additional forces directly to the base 239, providing a different force range for the force sensing device 10.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A force sensing device (1; 10; 19) comprising
a housing tip (21; 210; 219);
a plurality of sensors (31; 310; 319) stationarily spaced from each other; and
a spring element (4; 40; 49) connecting the housing tip (21; 210; 219) to the sensors (31; 310; 319) such that moving the housing tip (21; 210; 219) and the sensors (31; 310; 319) relative to each other causes a resilient deformation of the spring element (4; 40; 49); wherein
the spring element (4; 40; 49) is configured to arrange the housing tip (21; 210; 219) in a zero position relative to the sensors (31; 310; 319) by a spring force of the spring element (4; 40; 49),
a spring tensioning structure (5; 50; 59) configured to adapt the spring force of the spring element (4; 40; 49) in order that the range of forces to be detectable by the force sensing device (1; 10; 19) can be adjusted, **characterized in that** the force sensing device (1; 10; 19) can efficiently be prepared for different conditions and applications.

2. The force sensing device (1; 10; 19) of claim 1, wherein plurality of sensors comprises three sensors, wherein preferably the sensors (31; 310; 319) are unidirectional sensors (31; 310; 319), wherein further the sensors (31; 310; 319) are force sensors (31; 310; 319).

3. The force sensing device (1; 10; 19) of claim 2, comprising a plurality of sensor actuators (6), each associated to one of the force sensors (31; 310; 319), wherein the sensor actuators (6) are coupled to the housing tip (21; 210; 219) such that moving the housing tip (21; 210; 219) relative to the force sensors (31; 310; 319) out of the zero position causes at least one of the sensor actuators (6) to act on the associated force sensor (31; 310; 319), wherein preferably the sensor actuators (6) are stationarily mounted to the housing tip (21; 210; 219) and wherein preferably each of the sensor actuators (6) is adjustable in its extension towards the associated sensor (31; 310; 319).

4. The force sensing device (1; 10; 19) of any one of the preceding claims, comprising a preferably flexible sensor support plate (32; 320; 329) on which the sensors (31; 310; 319) are mounted, wherein preferably the sensor support plate (32; 320; 329) is a printed circuit board wherein the sensor support plate (32; 320; 329) is essentially ring-shaped, wherein preferably the sensors (31; 310; 319) are regularly distributed about a central hole of the ring-shaped sensor support plate (32; 320; 329).

5. The force sensing device (1; 10; 19) of any one of the preceding claims, wherein the spring tensioning structure (5; 50; 59) comprises a screw member (51; 510) and a screw socket (52; 520) such that by screwing the screw member (51; 510) into or out of the screw socket (52; 520) the spring force of the spring element (4; 40; 49) is adapted, wherein preferably the screw member (51; 510) extends along the spring element (4; 40; 49), wherein preferably the spring element (4; 40; 49) comprises a helical spring (4; 40; 49) being connected to the sensors (31; 310; 319) at one of its longitudinal ends and connected to the housing tip (21; 210; 219) at the other one of its longitudinal end .

6. The force sensing device (1; 10; 19) of any one of the preceding claims, comprising a safety structure (215, 221; 2150, 2310) limiting a maximum movement of the housing tip (21; 210; 219) and the sensors (31; 310; 319) relative to each other.

7. The force sensing device (1; 10; 19) of any one of the preceding claims, comprising a guiding structure (215, 221; 2150, 2310) predefining possible movements of the housing tip (21; 210; 219) and the sensors (31; 310; 319) relative to each other, wherein preferably the guiding structure (215, 221; 2150, 2310) is configured to prevent rotational movements of the housing tip (21; 210; 219) and the sensors (31; 310; 319) about a longitudinal axis of the force sensing device (1; 10; 19) relative to each other, wherein preferably the guiding structure (215, 221; 2150, 2310) is configured to limit lateral shifting of the housing tip (21; 210; 219) and the sensors (31; 310; 319) relative to each other and wherein preferably the guiding structure (215, 221; 2150, 2310) is configured to limit the movements of the housing tip (21; 210; 219) and the sensors (31; 310; 319) relative to each other to five degrees of freedom.

8. The force sensing device (1; 10; 19) of claim 6 or 7, wherein the safety structure (215, 221; 2150, 2310) and the guiding structure (215, 221; 2150, 2310) are embodied by at least one projection (215; 2150; 2159) stationary to one of the housing tip (21; 210; 219) or the sensors (31; 310; 319) and at least one corresponding recess (221; 2310; 2319) stationary to the other one of the housing tip (21; 210; 219) and the sensors (31; 310; 319), the at last one recess (221; 2310; 2319) receiving the at least one projection (215; 2150; 2159).

9. The force sensing device (1; 10; 19) of any one of the preceding claims, having a distal end and a proximal end, wherein the sensors (31; 310; 319) are configured to sense along an axis extending between the distal and proximal ends of the force sensing device (1; 10; 19).

10. A medical endodevice comprising an elongated liaising structure with a distal end and a force sensing device (1; 10; 19) according to any one of the preceding claims, wherein the force sensing device (1; 10; 19) is mounted to the distal end of the elongated liaising structure such that the housing tip (21; 210; 219) forms a tip of the medical endodevice, wherein the liaising structure comprises an articulated portion.

11. The medial endodevice of claim 10, wherein the articulated portion of the elongated liaising structure comprises a plurality of bodies and angle sensors, each body being tiltably connected to another one of the bodies and each angle sensor being configured to determine an angle between two connected bodies, wherein preferably each body is connected to another one of the bodies to be tiltable exclusively about one single axis, wherein further the elongated liaising structure comprises a force/torque sensor unit being proximally spaced from the distal end.

12. The medical endodevice of claim 11, wherein the articulated portion of the elongated liaising structure has a distal end side and a proximal end side, the force sensing device (1; 10; 19) is located distally of the articulated portion and the force/torque sensor unit is located proximally of the articulated portion.

13. The medical endodevice of claim 11 or 12, wherein the force sensor unit of the elongated liaising structure comprises
three unidirectional shaft force sensors stationarily spaced from each other;
three shaft sensor actuators, each associated to one of the three shaft force sensors; and
a shaft spring element connecting the articulated portion of the elongated liaising structure to the three shaft force sensors such that moving the articulated portion and the shaft force sensors relative to each other causes a resilient deformation of the shaft spring element, wherein
the shaft spring element is configured to arrange the articulated portion in a zero position relative to the shaft force sensors by a spring force; and
the shaft sensor actuators are coupled to the articulated portion such that moving the articulated portion relative to the shaft force sensors out of the zero position causes at least one of the shaft sensor actuators to act on the associated shaft force sensor.

14. An in vitro process of detecting a type of tissue, comprising
obtaining a medical endodevice according to any one of claims 11 to 13,
positioning the tip of the medical endodevice at a target tissue,
moving the tip of the medical endodevice along the target tissue,
obtaining sensor data generated by the sensors (31; 310; 319) of the force sensing device (1; 10; 19) of the medical endodevice, and
determining a stiffness of the target tissue by evaluating the obtained sensor data of the sensors (31; 310; 319) of the force sensing device (1; 10; 19).

15. The process of claim 14, comprising: obtaining sensor data generated by the force/torque sensor unit of the endodevice, and determining interactions along the endodevice by evaluating the sensor data of the force/torque sensor unit, and preferably calculating disturbances acting on the endodevice by comparing the sensor data of the force/torque sensor unit with the sensor data of the sensors (31; 310; 319) of the force sensing device (1; 10; 19).

## Patentansprüche

1. Kraftmessvorrichtung (1; 10; 19), umfassend
eine Gehäusespitze (21; 210; 219);
eine Vielzahl von Sensoren (31; 310; 319), die voneinander stationär beabstandet sind; und
ein Federelement (4; 40; 49), das die Gehäusespitze (21; 210; 219) mit den Sensoren (31; 310; 319) derart verbindet, dass ein Bewegen der Gehäusespitze (21; 210; 219) und der Sensoren (31; 310; 319) relativ zueinander eine elastische Verformung des Federelements (4; 40; 49) veranlasst; wobei
das Federelement (4; 40; 49) konfiguriert ist, um die Gehäusespitze (21; 210; 219) durch eine Federkraft des Federelements (4; 40; 49) in einer Nullposition relativ zu den Sensoren (31; 310; 319) anzuordnen,
eine Federspannstruktur (5; 50; 59), die konfiguriert ist, um die Federkraft des Federelements (4; 40; 49) anzupassen, damit der Bereich der Kräfte eingestellt werden kann, die durch die Kraftmessvorrichtung (1; 10; 19) erfassbar ist, **dadurch gekennzeichnet, dass** die Kraftmessvorrichtung (1; 10; 19) für unterschiedliche Bedingungen und Anwendungen effizient vorbereitet werden kann.

2. Kraftmessvorrichtung (1; 10; 19) nach Anspruch 1, wobei die Vielzahl von Sensoren drei Sensoren umfasst, wobei vorzugsweise die Sensoren (31; 310; 319) unidirektionale Sensoren (31; 310; 319) sind, wobei ferner die Sensoren (31; 310; 319) Kraftsensoren (31; 310; 319) sind.

3. Kraftmessvorrichtung (1; 10; 19) nach Anspruch 2, umfassend eine Vielzahl von Sensoraktuatoren (6), die jeweils einem der Kraftsensoren (31; 310; 319) zugeordnet sind, wobei die Sensoraktuatoren (6) mit der Gehäusespitze (21; 210; 219) derart gekoppelt sind, dass das Bewegen der Gehäusespitze (21; 210; 219) relativ zu den Kraftsensoren (31; 310; 319) aus der Nullposition heraus mindestens einen der Sensoraktuatoren (6) veranlasst, auf den zugeordneten Kraftsensor (31; 310; 319) einzuwirken, wobei vorzugsweise die Sensoraktuatoren (6) an der Gehäusespitze (21; 210; 219) stationär montiert sind und wobei vorzugsweise jeder der Sensoraktuatoren (6) in seiner Erstreckung zu dem zugehörigen Sensor (31; 310; 319) hin einstellbar ist.

4. Kraftmessvorrichtung (1; 10; 19) nach einem der vorhergehenden Ansprüche, umfassend eine vorzugsweise flexible Sensorträgerplatte (32; 320; 329), auf der die Sensoren (31; 310; 319) montiert sind, wobei vorzugsweise die Sensorträgerplatte (32; 320; 329) eine Leiterplatte ist, wobei die Sensorträgerplatte (32; 320; 329) im Wesentlichen ringförmig ist, wobei vorzugsweise die Sensoren (31; 310; 319) regelmäßig um ein zentrales Loch der ringförmigen Sensorträgerplatte (32; 320; 329) herum verteilt sind.

5. Kraftmessvorrichtung (1; 10; 19) nach einem der vorhergehenden Ansprüche, wobei die Federspannstruktur (5; 50; 59) ein Schraubenelement (51; 510) und eine Schraubfassung (52; 520) derart umfasst, dass durch Schrauben des Schraubenelements (51; 510) in die Schraubfassung (52; 520) hinein oder aus dieser heraus die Federkraft des Federelements (4; 40; 49) angepasst wird, wobei vorzugsweise sich das Schraubenelement (51; 510) entlang des Federelements (4; 40; 49) erstreckt, wobei vorzugsweise das Federelement (4; 40; 49) eine Schraubenfeder (4; 40; 49) umfasst, die an einem ihrer Längsenden mit den Sensoren (31; 310; 319) verbunden ist und an dem anderen ihrer Längsenden mit der Gehäusespitze (21; 210; 219) verbunden ist.

6. Kraftmessvorrichtung (1; 10; 19) nach einem der vorhergehenden Ansprüche, umfassend eine Sicherheitsstruktur (215, 221; 2150, 2310), die eine maximale Bewegung der Gehäusespitze (21; 210; 219) und der Sensoren (31; 310; 319) relativ zueinander begrenzt.

7. Kraftmessvorrichtung (1; 10; 19) nach einem der vorhergehenden Ansprüche, umfassend eine Führungsstruktur (215, 221; 2150, 2310), die mögliche Bewegungen der Gehäusespitze (21; 210; 219) und der Sensoren (31; 310; 319) relativ zueinander vordefiniert, wobei vorzugsweise die Führungsstruktur (215, 221; 2150, 2310) konfiguriert ist, um Drehbewegungen der Gehäusespitze (21; 210; 219) und der Sensoren (31; 310; 319) um eine Längsachse der Kraftmessvorrichtung (1; 10; 19) herum relativ zueinander zu verhindern, wobei vorzugsweise die Führungsstruktur (215, 221; 2150, 2310) konfiguriert ist, um eine seitliche Verschiebung der Gehäusespitze (21; 210; 219) und der Sensoren (31; 310; 319) relativ zueinander zu begrenzen und wobei vorzugsweise die Führungsstruktur (215, 221; 2150, 2310) konfiguriert ist, um die Bewegungen der Gehäusespitze (21; 210; 219) und der Sensoren (31; 310; 319) relativ zueinander auf fünf Freiheitsgrade zu begrenzen.

8. Kraftmessvorrichtung (1; 10; 19) nach Anspruch 6 oder 7, wobei die Sicherheitsstruktur (215, 221; 2150, 2310) und die Führungsstruktur (215, 221; 2150, 2310) durch mindestens einem Vorsprung (215; 2150; 2159), der an einem von der Gehäusespitze (21; 210; 219) oder den Sensoren (31; 310; 319) stationär ist, und mindestens eine entsprechende Aussparung (221; 2310; 2319) verkörpert ist, die an der dem anderen von der Gehäusespitze (21; 210; 219) oder den Sensoren (31; 310; 319) stationär ist, wobei die mindestens eine Aussparung (221; 2310; 2319) den mindestens einen Vorsprung (215; 2150; 2159) aufnimmt.

9. Kraftmessvorrichtung (1; 10; 19) nach einem der vorhergehenden Ansprüche, die ein distales und ein proximales Ende aufweist, wobei die Sensoren (31; 310; 319) konfiguriert sind, um entlang einer Achse zu messen, die sich zwischen dem distalen und dem proximalen Ende der Kraftmessvorrichtung (1; 10; 19) erstreckt.

10. Medizinische Endovorrichtung, umfassend eine längliche Verknüpfungsstruktur mit einem distalen Ende und eine Kraftmessvorrichtung (1; 10; 19) nach einem der vorstehenden Ansprüche, wobei die Kraftmessvorrichtung (1; 10; 19) an dem distalen Ende der länglichen Verknüpfungsstruktur derart montiert ist, dass die Gehäusespitze (21; 210; 219) eine Spitze der medizinischen Endovorrichtung ausbildet, wobei die Verknüpfugsstruktur einen Gelenkabschnitt umfasst.

11. Medizinische Endovorrichtung nach Anspruch 10, wobei der Gelenkabschnitt der länglichen Verknüpfungsstruktur eine Vielzahl von Körpern und Winkelsensoren umfasst, wobei jeder Körper mit einem anderen der Körper kippbar verbunden ist und jeder Winkelsensor konfiguriert ist, um einen Winkel zwischen zwei verbundenen Körpern zu bestimmen, wobei vorzugsweise jeder Körper mit einem anderen der Körper verbunden ist, um ausschließlich um eine einzige Achse herum kippbar zu sein, wobei die längliche Verknüpfungsstruktur ferner eine Kraft-/Drehmomentsensoreinheit umfasst, die von dem distalen Ende proximal beabstandet ist.

12. Medizinische Endovorrichtung nach Anspruch 11, wobei der Gelenkabschnitt der länglichen Verknüpfungsstruktur eine distale Endseite und eine proximale Endseite aufweist, wobei sich die Kraftmessvorrichtung (1; 10; 19) distal von dem Gelenkabschnitt befindet und sich die Kraft-/Drehmomentsensoreinheit proximal von dem Gelenkabschnitt befindet.

13. Medizinische Endovorrichtung nach Anspruch 11 oder 12, wobei die Kraftsensoreinheit der länglichen Verknüpfungsstruktur umfasst
drei unidirektionale Wellenkraftsensoren, die voneinander stationär beabstandet sind;
drei Wellensensoraktuatoren, die jeweils einem der drei Wellenkraftsensoren zugeordnet sind; und
ein Wellenfederelement, das den Gelenkabschnitt der länglichen Verknüpfungsstruktur mit den drei Wellenkraftsensoren derart verbindet, dass das Bewegen des Gelenkabschnitts und der Wellenkraftsensoren relativ zueinander eine elastische Verformung des Wellenfederelements veranlasst, wobei
das Wellenfederelement konfiguriert ist, um den Gelenkabschnitt durch eine Federkraft in einer Nullposition relativ zu den Wellenkraftsensoren anzuordnen; und
die Wellensensoraktuatoren mit dem Gelenkabschnitt derart gekoppelt sind, dass das Bewegen des Gelenkabschnitts relativ zu den Wellenkraftsensoren aus der Nullposition heraus mindestens einen der Wellensensoraktuatoren veranlasst, auf den zugehörigen Wellenkraftsensor einzuwirken.

14. In-vitro-Verfahren zum Erfassen einer Gewebeart, umfassend
Erhalten einer medizinischen Endovorrichtung nach einem der Ansprüche 11 bis 13,
Positionieren der Spitze der medizinischen Endovorrichtung an einem Zielgewebe,
Bewegen der Spitze der medizinischen Endovorrichtung entlang des Zielgewebes,
Erhalten von Sensordaten, die durch die Sensoren (31; 310; 319) der Kraftmessvorrichtung (1; 10; 19) der medizinischen Endovorrichtung erzeugt werden, und
Bestimmen einer Steifigkeit des Zielgewebes durch Auswerten der erhaltenen Sensordaten der Sensoren (31; 310; 319) der Kraftmessvorrichtung (1; 10; 19).

15. Verfahren nach Anspruch 14, umfassend: Erhalten von Sensordaten, die durch die Kraft-/Drehmomentsensoreinheit der Endovorrichtung erzeugt werden, und Bestimmen von Wechselwirkungen entlang der Endovorrichtung durch Auswerten der Sensordaten der Kraft-/Drehmomentsensoreinheit, und vorzugsweise Berechnen von Störungen, die auf die Endovorrichtung einwirken, durch Vergleichen der Sensordaten der Kraft-/Drehmomentsensoreinheit mit den Sensordaten der Sensoren (31; 310; 319) der Kraftmessvorrichtung (1; 10; 19).

## Revendications

1. Dispositif de détection de force (1 ; 10 ; 19) comprenant
un embout de boîtier (21 ; 210 ; 219) ;
une pluralité de capteurs (31 ; 310 ; 319) stationnairement espacés les uns des autres ; et
un élément de ressort (4 ; 40 ; 49) reliant l'embout de boîtier (21 ; 210 ; 219) aux capteurs (31 ; 310 ; 319) de telle sorte que le déplacement de l'embout de boîtier (21 ; 210 ; 219) et des capteurs (31 ; 310 ; 319) les uns par rapport aux autres provoque une déformation élastique de l'élément de ressort (4 ; 40 ; 49) ; dans lequel
l'élément de ressort (4 ; 40 ; 49) est conçu pour disposer l'embout de boîtier (21 ; 210 ; 219) en position zéro par rapport aux capteurs (31 ; 310 ; 319) par une force de ressort de l'élément de ressort (4 ; 40 ; 49),
une structure de tension de ressort (5 ; 50 ; 59) conçue pour adapter la force de ressort de l'élément élastique (4 ; 40 ; 49) afin que la plage de forces détectables par le dispositif de détection de force (1 ; 10 ; 19) peut être réglé, **caractérisé en ce que** le dispositif de détection de la force (1 ; 10 ; 19) peuvent être préparés efficacement pour différentes conditions et applications.

2. Dispositif de détection de force (1 ; 10 ; 19) selon la revendication 1, dans lequel la pluralité de capteurs comprend trois capteurs, dans lequel, de préférence, les capteurs (31 ; 310 ; 319) sont des capteurs unidirectionnels (31 ; 310 ; 319), dans lequel les capteurs (31 ; 310 ; 319) sont des capteurs de force (31 ; 310 ; 319).

3. Dispositif de détection de force (1 ; 10 ; 19) selon la revendication 2, comprenant une pluralité d'actionneurs de capteurs (6), chacun associé à l'un des capteurs de force (31 ; 310 ; 319), dans lequel les actionneurs de capteurs (6) sont accouplés à l'embout de boîtier (21 ; 210 ; 219) de telle sorte que le déplacement de l'embout de boîtier (21 ; 210 ; 219) par rapport aux capteurs de force (31 ; 310 ; 319) hors de la position zéro provoque l'action d'au moins un des actionneurs de capteur (6) sur le capteur de force associé (31 ; 310 ; 319), dans lequel les actionneurs de capteurs (6) sont de préférence montés de manière fixe sur l'extrémité du boîtier (21 ; 210 ; 219) et dans lequel, de préférence, chacun des actionneurs de capteur (6) est réglable dans son extension vers le capteur associé (31 ; 310 ; 319).

4. Dispositif de détection de force (1 ; 10 ; 19) selon l'une quelconque des revendications précédentes, comprenant une plaque de support de capteur de préférence flexible (32 ; 320 ; 329) sur laquelle les capteurs (31 ; 310 ; 319) sont montés, dans lequel, de préférence, la plaque de support de capteur (32 ; 320 ; 329) est une carte de circuit imprimé, dans lequel la plaque de support de capteur (32 ; 320 ; 329) est essentiellement en forme d'anneau, dans lequel, de préférence, les capteurs (31 ; 310 ; 319) sont régulièrement répartis autour d'un trou central de la plaque de support de capteur en forme d'anneau (32 ; 320 ; 329).

5. Dispositif de détection de force (1 ; 10 ; 19) selon l'une quelconque des revendications précédentes, dans lequel la structure de tension de ressort (5 ; 50 ; 59) comprend un élément de vis (51 ; 510) et une douille à vis (52 ; 520) de telle sorte qu'en vissant l'élément de vis (51 ; 510) dans ou hors de la douille à vis (52 ; 520) et la force de ressort de l'élément élastique (4 ; 40 ; 49) est adapté, dans lequel, de préférence, l'élément de vis (51 ; 510) s'étend le long de l'élément de ressort (4 ; 40 ; 49), dans lequel, de préférence, l'élément de ressort (4 ; 40 ; 49) comprend un ressort hélicoïdal (4 ; 40 ; 49) étant relié aux capteurs (31 ; 310 ; 319) à l'une de ses extrémités longitudinales et relié à l'embout de boîtier (21 ; 210 ; 219) à l'autre de son extrémité longitudinale.

6. Dispositif de détection de force (1 ; 10 ; 19) selon l'une quelconque des revendications précédentes, comprenant une structure de sécurité (215, 221 ; 2150, 2310) limitant un déplacement maximal de l'embout de boîtier (21 ; 210 ; 219) et des capteurs (31 ; 310 ; 319) les uns par rapport aux autres.

7. Dispositif de détection de force (1 ; 10 ; 19) selon l'une quelconque des revendications précédentes, comprenant une structure de guidage (215, 221 ; 2150, 2310) prédéfinissant les déplacements possibles de l'embout de boîtier (21 ; 210 ; 219) et des capteurs (31 ; 310 ; 319) les uns par rapport aux autres, dans lequel, de préférence, la structure de guidage (215, 221 ; 2150, 2310) est conçue pour empêcher les déplacements de rotation de l'embout de boîtier (21 ; 210 ; 219) et des capteurs (31 ; 310 ; 319) autour d'un axe longitudinal du dispositif de détection de force (1 ; 10 ; 19) les uns par rapport à aux autres, dans lequel, de préférence, la structure de guidage (215, 221 ; 2150, 2310) est conçue pour limiter le déplacement latéral de l'embout de boîtier (21 ; 210 ; 219) et des capteurs (31 ; 310 ; 319) les uns par rapport aux autres et dans lequel, de préférence, la structure de guidage (215, 221 ; 2150, 2310) est conçue pour limiter les déplacements de l'embout de boîtier (21 ; 210 ; 219) et des capteurs (31 ; 310 ; 319) les uns par rapport aux autres à cinq degrés de liberté.

8. Dispositif de détection de force (1 ; 10 ; 19) selon la revendication 6 ou 7, dans lequel la structure de sécurité (215, 221 ; 2150, 2310) et la structure de guidage (215, 221 ; 2150, 2310) sont incarnées par au moins une projection (215 ; 2150 ; 2159) stationnaire à l'un de l'embout de boîtier (21 ; 210 ; 219) ou les capteurs (31 ; 310 ; 319) par l'au moins un évidement correspondant (221 ; 2310 ; 2319) stationnaire à l'autre embout de boîtier (21 ; 210 ; 219) et des capteurs (31 ; 310 ; 319), l'au moins un évidement (221 ; 2310 ; 2319) recevant l'au moins une saillie (215 ; 2150 ; 2159).

9. Dispositif de détection de force (1 ; 10 ; 19) selon l'une quelconque des revendications précédentes, ayant une extrémité distale et une extrémité proximale, dans lequel les capteurs (31 ; 310 ; 319) sont conçus pour détecter le long d'un axe s'étendant entre les extrémités distale et proximale du dispositif de détection de force (1 ; 10 ; 19).

10. Dispositif terminal médical comprenant une structure de liaison allongée avec une extrémité distale et un dispositif de détection de force (1 ; 10 ; 19) selon l'une quelconque des revendications précédentes, dans lequel le dispositif de capteur de force (1 ; 10 ; 19) est monté à l'extrémité distale de la structure de liaison allongée de telle sorte que l'embout de boîtier (21 ; 210 ; 219) forme un embout de l'endodispositif médical, dans lequel la structure de liaison comprend une partie articulée.

11. Endodispositif médical selon la revendication 10, dans lequel la partie articulée de la structure de liaison allongée comprend une pluralité de corps et de capteurs d'angle, chaque corps étant relié de manière inclinable à un autre corps et chaque capteur d'angle étant configuré pour déterminer un angle entre deux corps reliés, dans lequel, de préférence, chaque corps est relié à un autre corps pour être inclinable exclusivement autour d'un seul axe, dans lequel en outre la structure de liaison allongée comprend une unité de capteur de force/couple étant proximalement espacée de l'extrémité distale.

12. Endodispositif médical selon la revendication 11, dans lequel la partie articulée de la structure de liaison allongée a un côté d'extrémité distal et un côté d'extrémité proximal, le dispositif de détection de force (1 ; 10 ; 19) est situé à l'extrémité distale de la partie articulée et le capteur de force/couple est situé à l'extrémité proximale de la partie articulée.

13. Endodispositif médical selon la revendication 11 ou 12, dans lequel l'unité de capteur de force de la structure de liaison allongée comprend
trois capteurs de force unidirectionnels d'arbre, espacés les uns des autres de façon stationnaire ;
trois actionneurs de capteur d'arbre, chacun associé à l'un des trois capteurs de force d'arbre ; et
un élément ressort de l'arbre reliant la partie articulée de la structure de liaison allongée aux trois capteurs de force de l'arbre, de sorte que le déplacement de la partie articulée et des capteurs de force de l'arbre les uns par rapport aux autres provoque une déformation élastique de l'élément ressort de l'arbre, dans lequel
l'élément de ressort d'arbre est conçu pour placer la partie articulée dans une position zéro par rapport aux capteurs de force de l'arbre grâce à une force de ressort ; et
les actionneurs de capteur d'arbre sont accouplés à la partie articulée de sorte que le déplacement de la partie articulée par rapport aux capteurs de force de l'arbre hors de la position zéro provoque l'action d'au moins un des actionneurs du capteur d'arbre sur le capteur de force d'arbre associé.

14. Procédé in vitro de détection d'un type de tissu, comprenant
l'obtention d'un endodispositif médical selon l'une quelconque des revendications 11 à 13,
le positionnement de l'embout de l'endodispositif médical sur un tissu cible, le déplacement de l'embout de l'endodispositif médical le long du tissu cible,
l'obtention des données de capteur générées par les capteurs (31 ; 310 ; 319) du dispositif de détection de force (1 ; 10 ; 19) de l'endodispositif médical, et
la détermination de la rigidité du tissu cible en évaluant les données obtenues des capteurs (31 ; 310 ; 319) du dispositif de détection de force (1 ; 10 ; 19).

15. Procédé selon la revendication 14, comprenant : l'obtention de données de capteur générées par l'unité de capteur de force/couple de l'appareil terminal, et la détermination des interactions le long de l'appareil terminal en évaluant les données de capteur de l'unité de capteur de force/couple, et de préférence, en calculant les perturbations agissant sur l'appareil terminal en comparant les données de capteur de l'unité de capteur de force/couple avec les données de capteur des capteurs (31 ; 310 ; 319) du dispositif de détection de force (1 ; 10 ; 19).
